# EUROPEAN PATENT APPLICATION

(11) **EP 4 763 993 A1**
(43) Date of publication of application: **24.06.2026**
(21) Application number: 24307128.9
(22) Date of filing: 17.12.2024
(51) Int. Cl.: C12Q 1/6806, C12Q 1/6837, C12Q 1/6841

(54) **ARRAYS AND METHODS FOR SPATIAL EPIGENOMIC PROFILING**

(71) Applicant: Commissariat à l'Energie Atomique et aux Energies Alternatives, 75015 Paris (FR); Université Evry Val d'Essonne, 91025 Evry-Courcouronnes (FR); Centre National de la Recherche Scientifique, 75016 Paris (FR)
(72) Inventor: MENDOZA-PARRA, Marco Antonio, 91000 EVRY (FR)
(74) Representative: Gevers & Orès

(57) **Abstract**

The present invention relates to the field of spatial epigenomic profiling. It concerns DNA arrays loaded with barcoded polynucleotides harboring a mosaic (MOS) sequence, methods and uses thereof enabling spatial epigenomic profiling.

## Description

The present invention relates to the field of spatial epigenomic profiling. It concerns DNA arrays loaded with barcoded polynucleotides harboring a mosaic (MOS) sequence, methods and uses thereof enabling spatial epigenomic landscaping.

Developments in spatially-resolved transcriptomics (SrT), and more recently on spatially-resolved epigenomics, are providing means to interrogate organ/tissue architecture from the angle of the gene programs defining their molecular complexity. Despite the proved performance of the available spatially-resolved omics technologies in a variety of tissue samples; there are still major challenges to address. Indeed, beyond the aspects related to the resolution of these technologies, their capacity to access to other type of molecular readouts (e.g. chromatin accessibility, epigenetics), their technical limitations issued from the way in which the biological specimens were preserved (formalin-fixed paraffin-embedded (FFPE) vs fresh frozen tissues), but also their current elevated costs, make these technologies applicable to few tissue sections, thus avoiding to generate multi-omic integrative views of their molecular complexity.

Spatial genomic landscaping is particularly advantageous in situations wherein technologies based on "spatial transcriptomics" do not work because RNA is destroyed, since it allows to infer gene expression by looking at the promoter epigenetics status. Furthermore, epigenetics profiling can also reveal genes that are physically repressed by the presence of histone modifications like H3K27me3 or H3K9me3; an aspect that cannot be addressed with spatial transcriptomics.

WO2022/147239 discloses a technology based on a microfluidic chip with parallel channels that is placed directly against a tissue sample on a slide and involves the use of a fusion protein of transposase and protein A (PA-Tn5) preloaded with a mosaic (MOS) sequence recognized by the transposase to capture the chromatin. This technology allows to perform a spatial cartography of tissue sections by capturing chromatin histone modification signatures. However, this approach shows some limits in that microfluidics system is particularly laborious and extremely costly.

In this context, there thus remains a genuine need for convenient and cost-efficient means for spatial epigenomic profiling.

The present invention is believed to meet such needs by providing a novel type of DNA array based on an innovative approach.

In an original way, the Inventors have developed a novel type of DNA arrays harboring barcoded DNA polynucleotides with MOS sequence, such that a transposase can be loaded *"in-situ".*

This technology is particularly advantageous since it allows to produce large number of DNA arrays at a low cost and to generate DNA arrays covering larger surfaces in a custom-made manner and still at reduced costs of production, in particular with the use of double-barcoded polynucleotides.

The spatial epigenomics strategy proposed in the present invention has been validated in fresh-frozen tissues, but also in decalcified FFPE conserved material (bone-related tissues), known to be incompatible with spatial transcriptomics assays because of RNA degradation. Furthermore, this technology has been used for performing spatial epigenomics profiling in mouse embryo samples, targeting several histone modifications assessed in consecutive sections and allowed to reveal relevant chromatin state signatures associated to either active, repressive or bivalent chromatin status across the tissue, providing a detailed molecular view of the chromatin regulation on the grounds of its physical location.

In a main aspect, the present invention thus relates to a DNA array exposing a set of barcoded DNA polynucleotides, wherein each polynucleotide is immobilized by one of its ends to the array, wherein:
each polynucleotide comprises:
   - a terminal region, at the end attached to the array,
   - a region for spatial barcoding,
   - a mosaic region (MOS), at the free end, said MOS region being recognized by a transposase,
and each distinct polynucleotide occupying a distinct position in a grid pattern corresponding to its spatial barcoding.

As used herein, the expression "terminal region" designates the part at the extremity of the barcoded DNA polynucleotide which is attached to the surface of the array. The terminal region according to the invention can be any known sequence. It can be used to copy and amplify the DNA fragment captured by the array.

As used herein, the expression "region for spatial barcoding" designates a region containing a unique sequence (or barcode) that can be used to distinguish a barcoded polynucleotide attached on the array from other barcoded polynucleotides attached on the same array. A region for spatial barcoding is thus associated with a particular location on the array and, thus, with a particular location in a biological sample immobilized on the array. The concept of "barcodes" and appending barcodes to polynucleotides are known to one of ordinary skill in the art.

Each location of the array (or "SpExel" for spatial epigenomics element, in analogy to pixel for picture element) corresponds to a unique spatially addressable barcoded polynucleotide (or a unique subset of spatially addressable barcoded polynucleotide) such that the location (or SpExel) can be identified based on that unique barcoded polynucleotide (or unique subset of barcoded polynucleotides).

As used herein, the expression "mosaic region" (or "MOS region") designates a region containing a specific transposase recognition sequence (or mosaic end sequence).

As used herein, the term "transposase" designates any member from the transposase family enzymes capable of cutting and binding DNA strands.

In some embodiments, the invention concerns a DNA array as defined above, wherein said transposase is selected from the group comprising Tn5, MuA, PiggyBac, and Sleeping Beauty, preferably Tn5.

The present invention can use any transposase that can accept a mosaic end sequence and fragment a target nucleic acid, attaching a transferred end, but not a non-transferred end.

In some embodiments, the invention concerns a DNA array as defined above, wherein the MOS region is a sequence of at least 19 nucleotides.

In some embodiments, the invention concerns a DNA array as defined above, wherein the MOS region comprises or consists of the sequence SEQ ID NO: 1 (CTGTCTCTTATACACATCT). SEQ ID NO: 1 corresponds to a mosaic end sequence that is specifically recognized by Tn5 transposase.

In some embodiments, the invention concerns a DNA array as defined above, wherein the region for spatial barcoding comprises:
- a region for spatial barcoding assigned to a specific row of the array (BCr), and
- a region for spatial barcoding assigned to a specific column of the array (BCc), and each distinct polynucleotide occupies a distinct position in a grid pattern corresponding to its BCr and BCc regions.

The use of combinations of two types of barcoding region per polynucleotide is particularly advantageous in that it drastically reduces the number of distinct barcodes required to cover the surface of the array. For example, in a 32x32 interstitially printed array (composed by 32x32x2 SpExels) 2,048 specific barcodes are required, but such an array can be produced by the combination of only 32 BCr barcodes and 64 BCc barcodes in the invention.

In some embodiments, each region for spatial barcoding contains a barcode of 8, 9, 10, 11, 12, 13, 14, 15, 16, 17, 18, 19 or 20 nucleotides.

In some embodiments, each region for spatial barcoding assigned to a specific row or a specific column contains a barcode of at least 8 nucleotides.

In some embodiments, each region for spatial barcoding assigned to a specific row or a specific column contains a barcode of 8, 9, 10, 11, 12, 13, 14, 15, 16, 17, 18, 19 or 20 nucleotides, preferably 8 nucleotides.

In some embodiments, each region for spatial barcoding assigned to a specific row or a specific column contains a barcode of only 8 nucleotides.

In some embodiments, the invention concerns a DNA array as defined above, wherein the region for spatial barcoding comprises:
- a BCr region,
- a BCc region,
- a bridge region located between BCr region and BCc region,
and each distinct polynucleotide occupies a distinct position in a grid pattern corresponding to its BCr and BCc regions.

In some embodiments, the invention concerns a DNA array as defined above, wherein the terminal region comprises a promoter.

The presence of a promoter sequence in the polynucleotide can be advantageous for particular uses because a promoter sequence can allow to artificially produce RNA to release it from the DNA array.

In another aspect, the invention also relates to method comprising:
(i) depositing a tissue section on top of a DNA array as defined in any one of claims ...,
(ii) delivering to the DNA array a primary antibody that specifically binds an epigenomic marker of interest, and possibly a secondary antibody that binds the first antibody, optionally said primary antibody and/or secondary antibody presenting a conjugated fluorophore,
(iii) delivering to the DNA array a fusion protein which comprises a moiety that binds the primary antibody, or the secondary antibody if present, and a moiety that is a transposase recognizing the MOS region of the barcoded DNA polynucleotides,
(iv) incubating the DNA array in conditions allowing the cleavage of the genomic DNA in the tissue section and its ligation with the MOS region of the barcoded DNA polynucleotides by the transposase, preferably in presence of MgCl₂,
(v) incubating the DNA array with a protease, preferably proteinase K.

As used herein, the term "tissue" designates any solid tissue. It can be adult tissue, embryonic tissue, or fetal tissue, preferably adult tissue. The tissue can be from any animal, preferably mammal, more preferably human. For example, a tissue sample may be obtained from mice, rats, rabbits, pigs, etc ...

In some embodiments, the tissue is fixed. Tissue fixation refers to the process of chemically preserving the natural state of a biological sample, for example, for subsequent histological analysis. Various fixation agents are routinely used, including, for example, formalin (e.g., formalin fixed paraffin embedded (FFPE) tissue), formaldehyde, paraformaldehyde and glutaraldehyde, any of which may be used herein to fix a biological sample. In such embodiments, the sample may be fixed before or after it is sectioned.

In some embodiments, the method of the invention further includes a step of permeabilization prior to depositing a tissue section on top of a DNA array. Thus, in some embodiments, the methods comprise delivering permeabilization reagents, e.g. detergents such as Triton-X 100 or Tween- 20, to the tissue.

In some embodiments, the invention concerns a method as defined above, further comprising:
(vi) copying the DNA captured by the barcoded DNA polynucleotides.

Copying the DNA captured by the barcoded DNA polynucleotides can be achieved by synthesizing a cDNA's complementary strand as detailed in the examples. For example, it can be achieved as follows: a step (a) of incubating the DNA array with a NaOH solution to produce DNA breaks, a step (b) of poly-C tailing by incubating the DNA array with a poly-C tailing mix containing a terminal transferase, dCTP and ddCTP, a step (c) of complementary strand extension by incubating the DNA array with a mix containing a Klenow polymerase, an oligonucleotide having a complementary sequence for the poly-C tailed sequence, an adapter sequence and dNTPs, and, then, a step (d) of recovering the synthesized complementary DNA strands by incubating the DNA array with a NaOH solution.

In some embodiments, the invention concerns a method as defined above, further comprising:
(vii) sequencing the DNA captured by the barcoded DNA polynucleotides to produce DNA reads.

Sequencing the DNA captured is achieved by using the copy of the DNA captured by the DNA polynucleotides, i.e. the synthesized complementary DNA strands, as a matrix. Preferably, it can be achieved by PCR amplification, followed by sequencing. Preferably, the step of sequencing is carried by next-generation sequencing (NGS) techniques, such as Illumina sequencing.

In some embodiments, the invention concerns a method as defined above, further comprising:
(viii) constructing a spatial map of the tissue sample by matching the barcoded nucleotides to corresponding sequencing reads.

In some embodiments, the invention concerns a method as defined above, further comprising:
(ix) identifying an anatomical location of the DNA captured by the barcoded DNA polynucleotides in the tissue sample.

In some embodiments, the invention concerns a method as defined above, wherein (i) further comprises imaging the sample to capture the position of the tissue sample on the DNA array.

In some embodiments, the invention concerns a method as defined above, wherein (ii) further comprises imaging the sample to capture the position of the epigenomic marker of interest.

In some embodiments, the imaging steps can be carried out by optical or fluorescence microscopy. Optical microscopy is particularly appropriate to image the position of the tissue section on the DNA array. Fluorescence microscopy is particularly appropriate to image conjugated antibodies or tagged probes.

In some embodiments, the invention concerns a method as defined above, wherein the fusion protein is composed by a transposase and a protein A moiety.

In some embodiments, the invention concerns a method as defined above, wherein the transposase is selected from the group comprising Tn5, MuA, PiggyBac, and Sleeping Beauty, preferably Tn5.

In some embodiments, the invention concerns a method as defined above, wherein the fusion protein is a Tn5 transposase, preferably a hyperactive Tn5, tethered to protein A.

In some embodiments, the invention concerns a method as defined above, wherein the primary antibody is selected from whole antibodies, Fab antibody fragments, F(ab')2antibody fragments, monospecific Fab2 fragments, bispecific Fab2 fragments, trispecific Fab3 fragments, single chain variable fragments (scFvs), bispecific diabodies, trispecific diabodies, scFv-Fc molecules, nanobodies, and minibodies.

In some embodiments, the invention concerns a method as defined above, wherein the epigenomic marker of interest is selected from the group consisting of H2AK5ac, H2AK9ac, H2BK120ac, H2BK12ac, H2BK15ac, H2BK20ac, H2BK5ac, H2Bub, H3, H3ac, H3K14ac, H3K18ac, H3K23ac, H3K23me2, H3K27me1, H3K27me2, H3K36ac, H3K36me1, H3K36me2, H3K4ac, H3K56ac, H3K79me1, H3K79me3, H3K9acS10ph, H3K9me2, H3S10ph, H3T11ph, H4, H4ac, H4K12ac, H4K16ac, H4K5ac, H4K8ac, H4K91ac, H3F3A, H3K27me3, H3K36me3, H3K4me1, H3K79me2, H3K9me1, H3K9me2, H3K9me3, H4K20me1, H2AFZ, H3K27ac, H3K4me2, H3K4me3, H3K9ac, and any other histone modification.

In another aspect, the invention also relates to the use of a DNA array as defined above for spatial mapping of an epigenomic marker of interest.

In another aspect, the invention also relates to a method of manufacturing a DNA array as defined above, comprising immobilizing DNA polynucleotides to the array, each polynucleotide comprising:
- a terminal region, at the end attached to the array,
- a region for spatial barcoding,
- a mosaic region (MOS), at the free end, said MOS region being recognized by a transposase,
and each distinct polynucleotide being immobilized at a distinct position in a grid pattern corresponding to its spatial barcoding.

The DNA polynucleotides can be attached to the array by UV irradiation.

In some embodiments, the invention concerns a method of manufacturing a DNA array as defined above, comprising:
(i) immobilizing single stranded DNA polynucleotides by their 5' end to the array, each polynucleotide comprising from 5' end to 3' end:
   - a terminal region,
   - a region for spatial barcoding assigned to a specific row of the array (BCr),
   - a bridge region,
      each polynucleotide being positioned at a distinct position in a grid pattern corresponding to its BCr region,
(ii) delivering at the surface of the array single stranded DNA polynucleotides, each polynucleotide comprising from 5' end to 3' end:
   - a mosaic region (MOS) being recognized by a transposase,
   - a region for spatial barcoding assigned to a specific column of the array (BCc),
   - a bridge region, which is complementary of the bridge region of the polynucleotides immobilized on the array,
      each polynucleotide being positioned a distinct position in a grid pattern corresponding to its BCc region,
(iii) hybridizing and elongating the polynucleotides by a DNA polymerase, preferably a T4 DNA polymerase.

The above embodiments based on the hybridization and elongation of two types of barcodes, BCr and BCc, are particularly advantageous since they involve combinations of distinct barcodes and can be carried out with a reduced number of distinct polynucleotides.

In another aspect, the invention also relates to a DNA polynucleotide comprising :
- a terminal region, at one end
- a region for spatial barcoding,
- a mosaic region (MOS), at the other end, said MOS region being recognized by a transposase.

In another aspect, the invention also relates to a set of two groups of DNA polynucleotides, wherein :
- the first group of DNA polynucleotides comprises from 5' end to 3' end:
   o a terminal region,
   o a region for spatial barcoding,
   ∘ a bridge region, and
- the second group of DNA polynucleotides comprises from 5' end to 3' end:
   o a mosaic region (MOS) being recognized by a transposase,
   o a region for spatial barcoding,
   o a bridge region, which is complementary of the bridge region of the DNA polynucleotide of the first group.

In another aspect, the invention also relates to a kit comprising at least:
- a DNA array as defined above,
- a DNA polynucleotide as defined above, or
- a set of two groups of DNA polynucleotides as defined in any one of claims ....

In some embodiments, the invention concerns a kit as defined above, further comprising an antibody, preferably an antibody that specifically binds an epigenomic marker of interest.

In some embodiments, the invention concerns a kit as defined above, further comprising at least one reagent selected from tissue fixation reagents, cDNA synthesis reagents, PCR reagents, and sequencing reagents.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

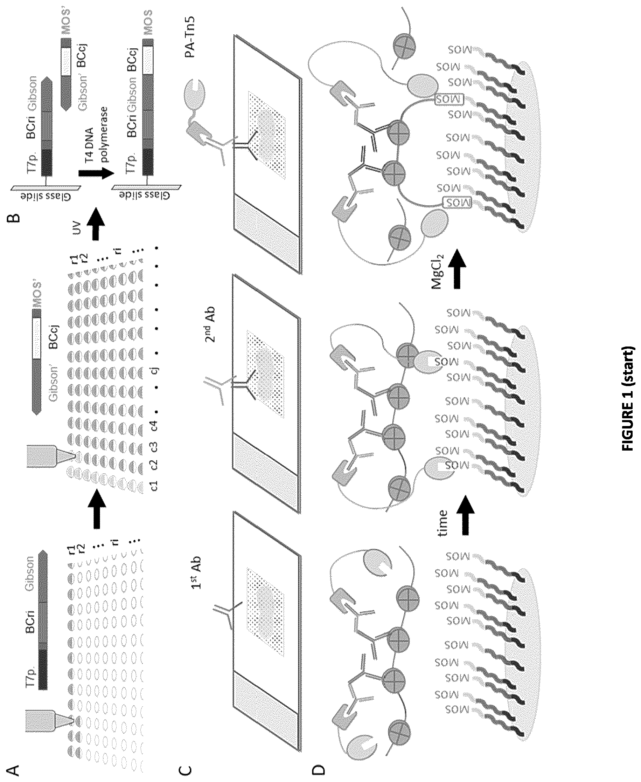

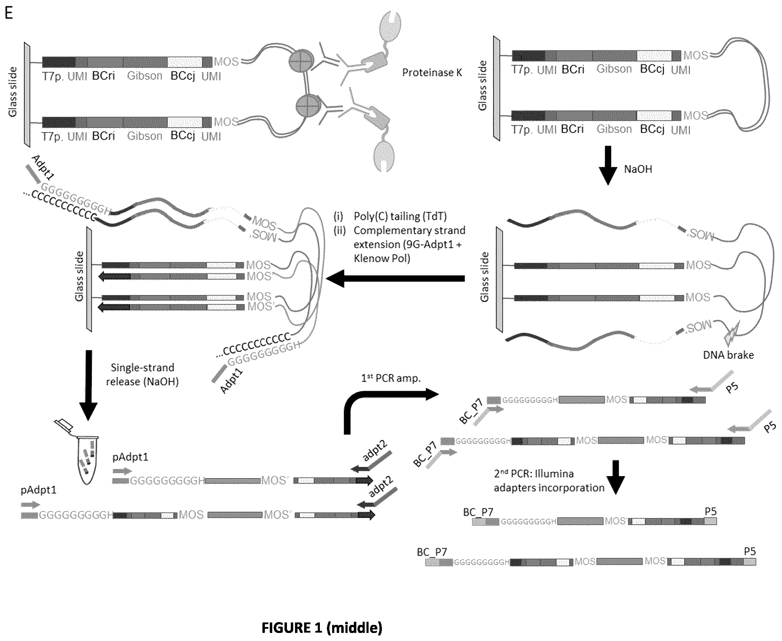

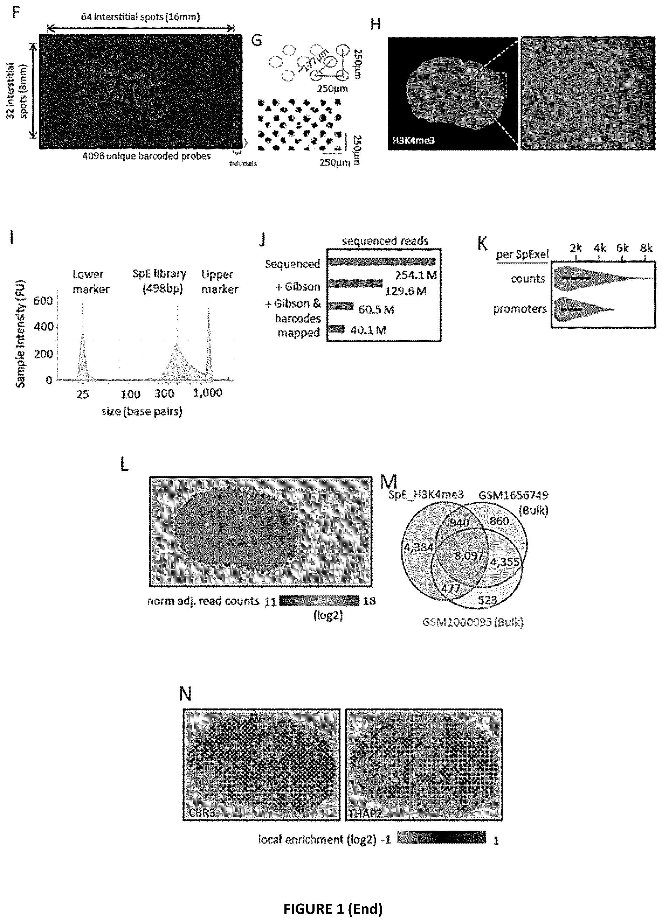

**Figure 1****. Principle behind the double-barcoded spatial epigenomics technology. (A)** DNA arrays are printed by depositing first probes in a row manner, followed by deposition of a second type of probes in a column manner. The first type of probes is composed by a T7 promoter (T7p) sequence, a unique molecular barcode associated to the row (BCr1, ...BCri) and bridge sequence called "Gibson". The second type of probes present a complementary Gibson sequence, a unique molecular barcode associated to the column (BCc1,...BCcj) and a complementary mosaic sequence (MOS'). **(B)** Since both probes were combined during printing, after UV irradiation for immobilization, hybridized probes are elongated with the T4 DNA polymerase, leading to a double-strand molecule presenting the mosaic sequence (MOS) at the free end of the probe. **(C)** After depositing a tissue section on top of the manufactured DNA array, a 1st antibody against the protein of interest is incubated, followed by a second antibody against the 1st antibody and finally incubated with the recombinant protein A-transposase PA-Tn5. **(D)** Scheme illustrating the loading of the Tn5 into the Mosaic sequence retrieved on the printed probes on the DNA array, followed by the chromatin cleavage induced by Magnesium chloride, leading to the formation of hairpin-like DNA structures. **(E)** Scheme illustrating the molecular biology steps required for generating a copy of the genomic DNA captured by the DNA probes, followed by their amplification for Illumina massive parallel DNA sequencing. **(F)** Micrograph displaying the scanning of a mouse brain coronal section deposited on top of a DNA array composed of 64x32 interstitial printed probes harboring the Mosaic sequence. Three rows of Cy3-labelled probes are visible at the border of the micrograph corresponding to fiducials used for defining the physical position of the tissue section across the 4096 printed probes. **(G)** micrograph displaying a bright-field scanning of a DNA array printed area where each of the printed probes are visible thanks to salt deposition. Thanks to the interstitial printing, the pitch distance resolution of the array is ~177microns. **(H)** Immunolabelling of the section displayed in (F) revealing the histone modification H3K4me3. The Inset magnification area reveals the nuclear staining visible through this labelling, as well as the differences in cell density across different parts of the mouse brain section. **(I)** Electropherogram displaying the spatial epigenomics (SpE) Illumina sequencing library obtained from the mouse brain section. **(J)** Number of sequenced reads and recovered at different stages of the primary bioinformatics analysis. **(K)** Violin plots displaying the number of read counts or promoter regions per SpExel (Spatial Epigenomics element, in analogy to Pixels: Picture elements). **(L)** Digital display corresponding to the number of adjusted read counts associated to the physical positions corresponding to the mouse brain section (heatmap displayed in logarithmic scale). **(M)** Venn-diagram displaying the number of promoters presenting H3K4me3 peaks (Macs2 P-value <1x10-5) in common between the spatial epigenomics map (SpE) and two bulk H3K4me3 ChIP-sequencing profiles (GSM1656749; GSM1000095). For this comparison, all mapped reads across the tissue were collapsed to generate a pseud-bulk profile. **(N)** Example of H3K4me3 enrichment signatures retrieved in the gene promoter region coding for the Carbonyl reductase 3 (Cbr3), or the Adenylyl cyclase 5 (Adcy5). Notice that CBR3 is preferentially over-expressed in the mouse brain basal ganglia, while THAP2 presented spatial signatures mainly concentrated around the *corpus callosum* structure.

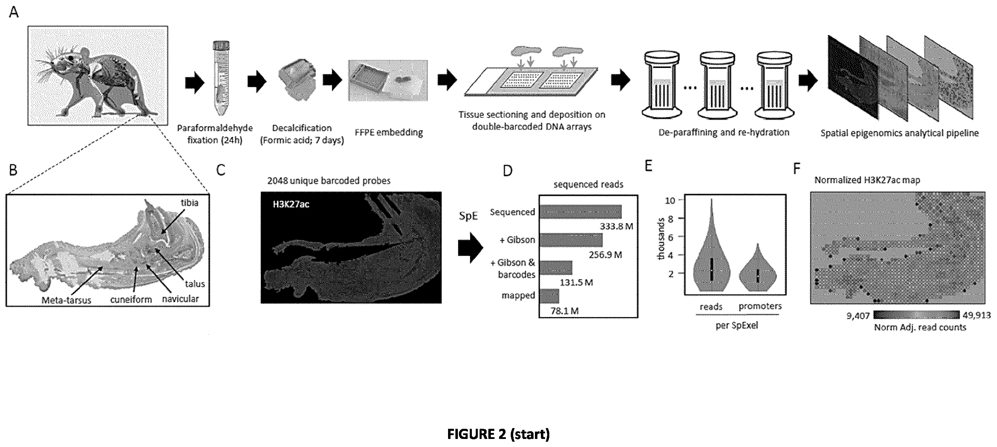

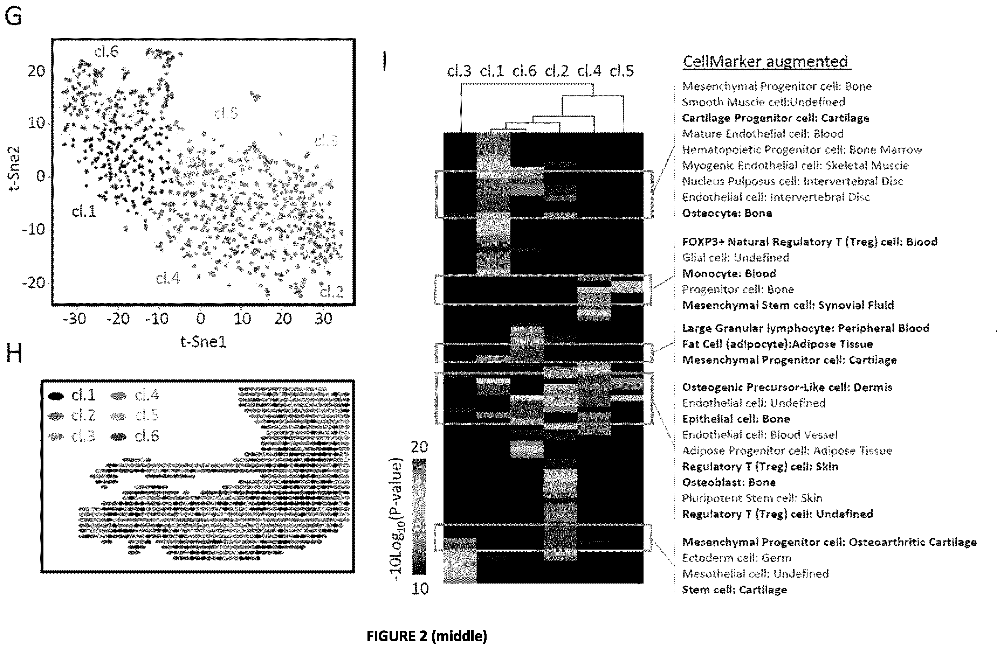

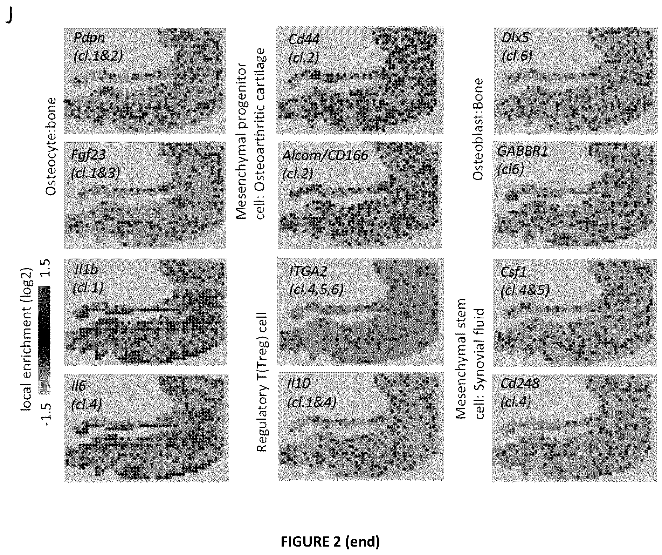

**Figure 2****. Spatial epigenomics assay in decalcified FFPE embedded tissues. (A)** Strategy for analyzing mice paw tissues modeling Rheumatoid arthritis (RA). **(B)** Representative mouse paw tissue section revealing the presence of the tibia, talus, navicular, cuneiform and meta-tarsus bone. **(C)** H3K27acethylation spatial epigenomics profiling performed on a mouse paw tissue section deposited on double-barcoded DNA array (64x32 printed probes). **(D)** Number of sequenced reads and recovered at different stages of the primary bioinformatics analysis. **(E)** Violin plots displaying the number of read counts or promoter regions per SpExel (Spatial Epigenomics element, in analogy to Pixels: Picture elements). **(F)** Digital view of the normalized and adjusted number of read counts associated to the physical location (SpExel) of the tissue section. **(G)** T-distributed stochastic neighbor embedding dimensionality reduction analysis (t-SNE) displaying the presence of 6 clusters of SpExels; **(H)** Clusters' projection on top of the physical position within the tissue. **(I)** Tissue-cell types gene marker association analysis performed per clusters identified in (G). Relevant tissue-cell types retrieved enriched per cluster are highlighted (boxes). **(J)** Example of gene promoters found enriched in H3K27ac read counts and associated to the aforementioned clusters. Cell type/tissue annotations indicated aside are collected from the CellMarker augmented database used for this analysis.

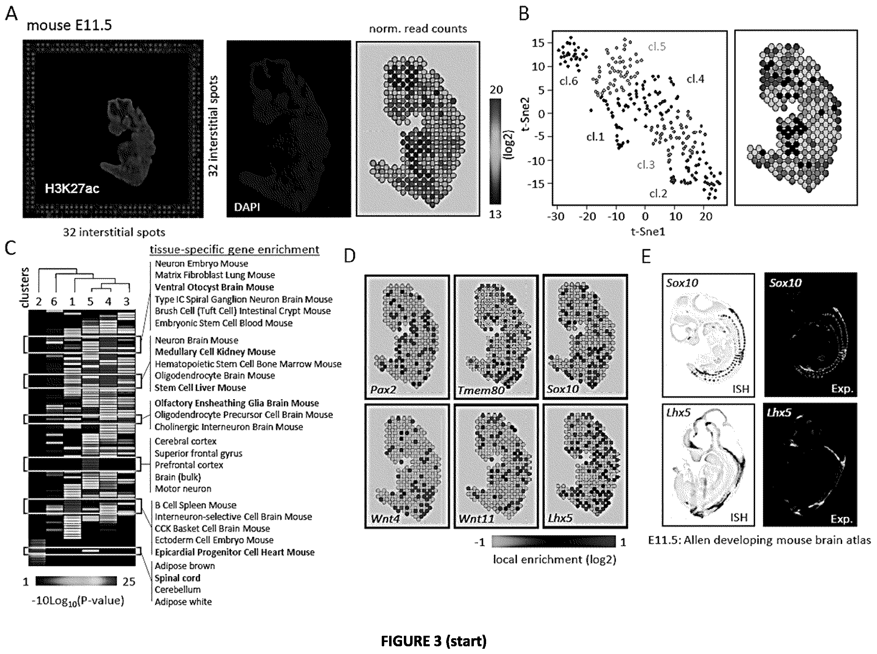

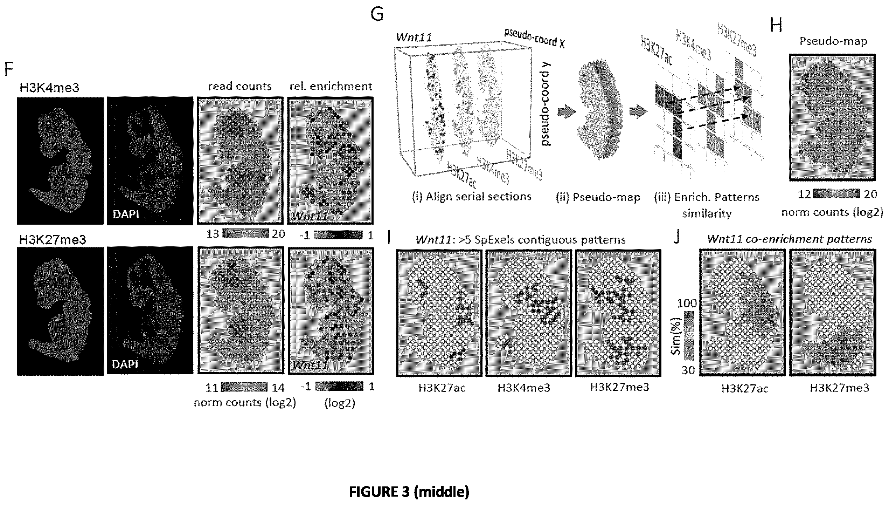

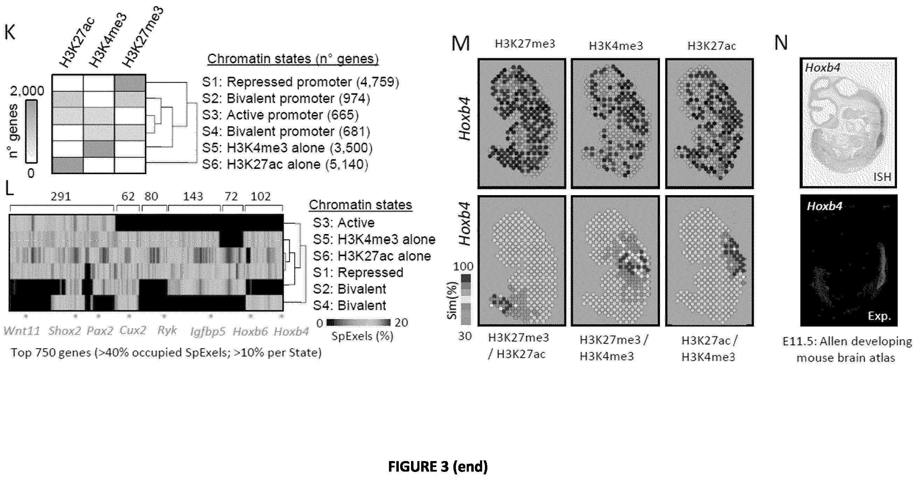

**Figure 3****. Inferring chromatin state transitions across the mouse embryo by integrating consecutive spatial epigenomic landscapes. (A)** Left panel: Scan of a DNA array (TRITC filter) hosting a cryosection of a mouse embryo (E11.5) and immuno-stained with an antibody targeting the histone modification H3K27acethylation (H3K27ac). Notice the presence of Cyanin-3 (Cy3)-labelled DNA probes delimiting the DNA array composed of 32x32 interstitially printed probes. Middle panel: DAPI staining; Right panel: Digital map displaying the normalized read counts captured per physical position (SpExel) across the mouse embryo section. **(B)** T-SNE analysis allowing to stratify SpExels in 6 different clusters. Right panel: Projection of the stratified clusters within the digital map. **(C)** Tissue-cell types gene marker association analysis performed per clusters identified in (B). **(D)** Local enrichment signatures associated to 6 gene promoters presenting H3K27ac over-represented counts. **(E)** *In situ* hybridization (ISH) and gene expression data (Allen mouse brain atlas) for the gene Sox10 and Lhx5, revealing its spatial signature, which is coherent with the H3K27ac digitized view displayed in D. **(F)** From left to right: Histone modifications immunostaining (TRITC filter: H3K4me3, H3K27me3); nuclei revealed by DAPI; digital map of the normalized read counts across the tissue section; local read counts enrichment associated to the gene *Wnt11.* **(G)** Strategy for interrogating changes in chromatin histone modification signatures across the embryo: (i) Overlay of all three histone modification maps; (ii) generate a pseudo map where SpExels of each digitized map are allocated to common pseudo-coordinates; (iii) Interrogate for contiguous enrichment patterns similarity within tissue section, but also across all three aligned sections. **(H)** Pseudo-map obtained from the overlay of all three-histone modification digital maps. **(I)** Promoter enrichment patterns associated to Wnt11 gene (>5 contigous SpExels) retrieved in either of the histone modification maps. **(J)** Spatial gene promoter's co-enrichment analysis for *Wnt11* in the H3K27ac or H3K27me3 digital map. SpExels colored in red correspond to the location of *Wnt11,* while others correspond to other gene promoters sharing a similar spatial pattern (Tanimoto similarity index). Notice that these two spatial gene promoter's co-enrichment maps present distinct spatial localizations. **(K)** Heatmap displaying the co-occurring promoter enrichment patterns between all three histone modifications. Six chromatin co-occurring states were identified, and functionally associated to either active, repressed, or bivalent promoters. Notice the presence of two states for which no functional association has been attributed H3K4me3, or H3K27ac alone). **(L)** Gene promoters presenting different chromatin co-occurring states across the tissue. **(M)** Top: Local read counts promoter enrichment for the gene Hoxb4 in either of the histone modification maps within the pseudo map. Bottom: Hoxb4 co-enrichment patterns revealing the presence of either bivalent (H3K27me3/H3K27ac or H3K27me3/H3K4me3) or promoter active regions (H3K27ac/H3K4me3). **(N)** *In situ* hybridization (ISH) and gene expression data (Allen mouse brain atlas) for the gene Hoxb4, revealing its spatial signature coherent with the H3K27ac digitized view displayed in M.

### EXAMPLES

### Materials and Methods

### DNA arrays manufacturing

DNA arrays for spatial epigenomics experiments were produced by depositing two types of complementary oligonucleotides, namely "Barcode for rows (BCr)" and "Barcode for columns (BCc)" hosting sequences. Double-barcoded DNA arrays for spatial transcriptomics were used.

The BCr oligonucleotide presents an amino C6 linker at the 5' extremity, followed by four G or C nucleotides (S), a T7 promoter sequence (GACTCGTAATACGACTCACTATAGGG, SEQ ID NO: 2), a unique molecular idenfitier (UMI: WSNNWSNNV), a molecular barcode (8 nucleotides) associated to the printed row in the DNA array and a 30 nucleotides long adapter sequence with a GC-content of 40%, (here named as Gibson sequence: ACATTGAAGAACCTG-TAGATAACTCGCTGT, SEQ ID NO: 3).

The BCc oligonucleotide has been modified for spatial epigenomics applications as following: At its 5' extremity, it presents a "CTGTCTCTTATACACATCT" (SEQ ID NO: 1) (herein called as Mosaic sequences or MOS), corresponding to the sequence recognized by the transposase Tn5. Then, BCc contains a unique molecular idenfitier, a molecular barcode (8 nucleotides) associated to the printed column in the DNA array, and a complementary Gibson sequence.

Oligonucleotides were diluted to 5 µM in presence of sciSPOT Oligos B1 solution (Scienion CBD-5421-50) in a 96 wells plate and stored at -20°C for long storage. DNA arrays were printed onto Superfrost Plus Adhesion Microscope Slides (J7800AMNZ Epredia). For mouse embryo assays, DNA arrays composed by 32x32 interstitially printed probes (2048 different probes in total, 177 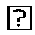 m pitch distance; ~100 µm printed spot) have been manufactured. For this, 32 BCr oligonucleotides presenting a unique molecular barcode were printed per row, by depositing ~250 pL (250 µm between contiguous spots) with a DNA pico-litter spotter (Scienion sciflexarrayer S3). Similarly, 32 different BCc oligonucleotides were printed per column, by spotting ~250 pL of each of them on top of the previoulsy prinded BCr nucleotides. Then, the same 32 BCr oligonucleotides were printed per row with a shifted position of 125 µm in both axis (i.e. interstitial printing), followed by the deposition of 32 different BCc oligonucleotides printed per column. In that manner a total 32 unique BCr (printed twice) and unique BCc oligonucleotides are required for reaching an array presenting 2,048 unique double-barcoded probes.

For mouse brain sections, it was generated 64x32 interstitially printed arrays (4096 different probes), where 32 BCr oligonucleotides presenting a unique molecular barcode were printed per row, other 32 different BCr oligonucleotides were printed per row with a shifted position of 125 µm in both axis (i.e. interstitial printing), followed by the deposition of 64 different BCc oligonucleotides printed per column. Finally, for mouse paws studies, a simpler DNA array printout was prodruced (64x32 DNA arrays) by depositing 32 BCr probes per row and 64 BCc probes per column (250 microns pitch distance, 100 microns spot diametter).

In all printout designs, fiducial borders were printed per DNA array, by adding three row/columns of spots with the used pitch distance (250 µm) by printing a BCr1 oligonucleotide presenting a Cy3-label at the 3'-extremity. During fiducial manufacturing, some spots were skipped in purpose for creating asymetry which will help for image scanning.

UV irradiation (254 nm; 5 min) was applied for crosslinking, followed by T4 DNA polymerase elongation (depending on the size of the printing area: 15 µL to 40 µL µL /printed region with coverslips: 0.06 U/µL T4 DNA Polymerase (New England Biolabs M0203L); 0.2 mM dNTPs (Life technologies R0141, R0151, R016, R0171) during 1 hour at 37°C). In order to evaluate the double-strand DNA elongation performance, a quality control assay was made within every batch production (18 slides per batch; 2 printed regions per glass slide for size areas of 8X16 mm (32x64 either linear or interstitially printed spots), or 3 printed areas for 8x8 mm arrays (2,048 spots)), by incorporating a dCTP-Cy3 nucleotide during the elongation (dATP/GTP/TTP at 0.2 mM, dCTP at 0.005 mM and dCTP-Cy3 at 0.002 mM (Cytiva PA53021)), and analyzed by fluorescent microscopy. After elongation, slides were washed with 0.1X SSC buffer (Merck S6639), then ddH₂O and finally spin dried (Labnet slide spinner C1303-T-230V, 4800 rpm). DNA array slides have been stored at +4°C in a sealed container.

### Fresh-frozen tissue cryosectioning

Tissue samples were cryosectionned (16 µm sections for mouse embryo tissue and and 20 µm sections for mouse brain WT tissue), collected between -15°C and -12°C then deposited on the DNA-arrays defined regions. Tissue sections were fixed with 1% Para-formaldheyde (PFA) (Life technologies 28908) in 1X PBS (Life technologies 70011-036) at room temperature during 30 minutes, then washed three times with 1X PBS.

### Mouse paws decalcification, formalin-fixed embedding, and sectioning

Mouse paws have been collected and fixed in 4% Paraformaldehyde (PFA) in 1X PBS for 24 hours. Then paws have been decalcified for 7 consecutive days in an +4°C refrigerated room on agitation. A maximum of 5 paws, each inside a cassette, are decalcified in 1L solution of 8,5g NaCl, 10% formol and 100mL of 99-100% glacial acetic acid. At mid incubation time, decalcification solution has been renewed and paws have been cut in half and put back into the cassette. Once decalcification is finished, paws have been rinsed in ddH₂0 to then be dehydrated and infiltrated with paraffin using an automated device (Novalix facilities) following this standard protocol: 70% ethanol, 2 washes of 70% ethanol, 96% ethanol, 100% ethanol, half and half mix of 100% ethanol with 100% xylene, empty the incubation chamber and final step of 100% xylene. Once dehydrated, each half paw has been paraffine embedded. All blocks of mouse paw FFPE can be stored at room temperature.

4µM thick sections around the Talus bone have been collected from those FFPE mouse paw blocks using a microtome. Paw FFPE sections were rehydrated and deparaffined following a standard protocols on an automated device (Novalix facilities).

### Histone modifications associated chromatin capturing in situ

Tissue sections were permeabilized during 1 hour at room temperature with 1% Triton X-100 (Merck 93443) in 1X PBS solution, then washed three times with 1X PBS followed by 5 minutes incubation at room temperature with 0.1M HCl for chromatin loosening, and washed three times with 1X PBS.

Afterwads, sections were covered during 1 hour at room temperature with a blocking and permeabilizing mix (10% Normal Donkey serum (NDS) (Merck D9663), 0.25% Triton X-100 (Merck 93443), 0.1% Bovine Serum Albumin (BSA) (Merck A9418), 2 mM EDTA (Life technologies AM9260G), 0.5 mM Spermidine (Life technologies 132740010), 1 tablet EDTA-free Protease Inibitor Cocktail (PIC) (Merck 11873580001)) prepared in 1X PBS. Then tissue were washed twice with 150 mM NaCl Buffer (20 mM Hepes-KOH 1M pH=7.2 (Merck 391338), 150 mM NaCl; 0.1% BSA, 0.5 mM Spermidine, 1 tablet PIC).

After that, sections were incubated with a solution containing 2 µg of a selected primary Antibody (anti-Histone H3 (tri methyl K4) antibody (Abcam ab213244); anti-Histone H3 (acethyl K27) antibody (Abcam ab177178); or anti-Histone H3 (tri methyl K27) antibody (Abcam ab 192985)), in 150 mM NaCl Buffer at room temperature during 1 hour, followed by washing twice with 150 mM NaCl Buffer. Then tissues were incubated at room temperature with a pre-incubated mix (2 hours) containing 1:1 molar ratio of unloaded fusion protein pA-Tn5 (in-house production (9.5 µM) prepared using the plasmid Addgene #124601, and the corresponding secondary Antibody (donkey anti-rabbit IgG antibody (Merck SAB3700932). The pre-incubated mix was complemented with either a conjugated Alexa-488 secondary Antibody (donkey anti-rabbit IgG antibody, Alexa Fluor 488 (Life technologies A-21206)) or a conjugated Alexa-555 secondary Antibody (donkey anti-rabbit IgG antibody, Alexa Fluor 555 (Abcam ab 150062)), added at 1/1,000 dilution.

The pre-incubated mix was used in a 10 times molar excess relative to the number of moles of the MOS sequence deposited per DNA array: for 2,048 spots it corresponds to 2,56.10⁻¹² moles; for 4,096 spots it corresponds to 5,12.10⁻¹² moles.

After 3 hours of incubation with the assembled mix, tissue sections were washed twice with 300 mM NaCl Buffer (20 mM Hepes-KOH 1M pH=7.2, 300 mM NaCl; 0.1 % BSA, 0.5 mM Spermidine, 1 tablet PIC) to remove PA-Tn5-antibodies in excess. Then, tissue sections were incubated during 5 minutes with DAPI (Life technologies 62248) 1/5,000 dilution in 1xPBS, then washed twice with PBS 1X.

DNA arrays were scanned under the TRICT filter to reveal the presence of the fiducial borders as well as the physical position of tissue sections on top of the printed DNA arrays. In case of the use of the conjugated Alexa-488 secondary Antibody, an additionnal scanning using FITC filter was done to reveal the localisation of the studied modification of histone. Finally, an additional scanning of the tissue using DAPI filter was done to observe nuclear localisation and to get cell density information. After imaging, sections were incubated overnight at 37°C with 10 mM MgCl₂ (Life technologies AM9530G) in 300 mM NaCl Buffer for *in situ* tagmentation.

Next day, tissue sections were incubated with a mix containing 2.25 µL of EDTA 0.5 M, 2.75 µL of 10% Sodium dodecyl sulfate (SDS) (Merck 71736) and 0.5 µL of 20 mg/mL Proteinase K (Life technologies 25530049) in 50 µL of ddH₂O overnight at 37 °C. The mix volume was ajusted depending of the slide printing area.

Finally, slides were washed under agitation (300 rpm) in containers containing: 100 mL of preheated buffer 1 (2X SSC and 0.1% SDS) during 15 minutes at 50°C, then 10 minutes with buffer 2 (0.2X SSC) at room temperature and 10 minutres with buffer 3 (0.1X SSC) at room temperature. At last the slides were washed in ddH₂O and spin-dried.

After that, DNA arrays were incubated with 0.1 M NaOH solution during 10 minutes at room temperature and then neutralized after removing liquid with a mix containig 100 µL of 0.1 M NaOH, 11.8 µL of 10X TE and 6.5 µL of 1.25 M acetic acid during 2-3 minutes. Finally, slides were washed within a falcon containing buffer 3 (0.1X SSC), then into a falcon containing ddH₂O and spin-dried.

### cDNA's complementary strand synthesis

DNA arrays were incubated with a poly-C tailing mix containing 0.6 U/µL terminal transferase (New England Biolabs M0315), 1X terminal transferase buffer (New England Biolabs BO315), 0.3X CoCl₂ (New England Biolabs B0252), 0.2 mM dCTP and 0.01 mM ddCTP during 35 minutes at 37°C, then 20 minutes at 70°C, and cooled at 12°C. To avoid evaporation, each printing area was closed by using sealing chambers (Life technologies AB-0576 (size 25 µL) or AB-0577 (size 65 µL)). Finally, slides were washed within a falcon containing buffer 3, and a falcon containing ddH₂O, prior spin-dry.

DNA arrays were then incubated with a mix containing: 0.1 U/µL Klenow exo- (New England Biolabs M0212), 0.2 mg/mL BSA, 1X NEB2 buffer (New England Biolabs B7002), 0.5 mM dNTPs and an oligonucleotide (1 µM) having a complementary sequence for the poly-C tailed sequence, as well as a 5'-extremity providing an adapter sequence (GTTCAGACGTGTGCTCTTCCGATCTGGGGGGGGGH, SEQ ID NO: 4). As in the preceding step to avoid evaporation, the reaction was performed by using sealing chambers.The following conditions were used: 47°C during 5 minutes (primer annealing), 37°C during 1 hour (extension), 10 minutes at 70°C (enzyme deactivation) and cooled et 12°C. In order to recover the synthesized complementary DNA strands, DNA arrays were incubated with 100 µL of 0.1 M NaOH solution during 10 minutes, then the liquid was collected and a second incubation with 0.1 M NaOH solution was repetead. The two fractions were collected together and neutralized (for 100 µL 0.1 M NaOH solution add 11.8 µL of 10X TE and 6.5 µL of 1.25 M acetic acid). DNA arrays were also neutralized with 100 µL 0.1 M NaOH solution mixed with 11.8 µL of 10X TE and 6.5µL of 1.25 M acetic acid, then washed with buffer 3 and ddH₂O and stored at 4 °C for eventual supplementary experiments.

The neutralized collected solution were precipitated with cold 100% ethanol (2X volume), 200 mM NaCl and 1 µL of 20 mg/mL glycogen (Merck 10901393001) and conserved at -20°C for at least 1 hour, then centrifuged at 12,500 rpm during 30 minutes, washed once with cold 70% ethanol and resuspended (after 30 minutes air drying) in 23 µL of ddH₂O.

### Ilumina sequencing library preparation

Half of the resuspended cDNA's complementary-probe material (12ul) was adjusted to 23ul with water, then mixed with: 25 µL of Q5 hot start high fidelity 2X master mix (New England Biolabs M0464L), 1 µL of adapter seq1 primer (GTTCAGACGTGTGCTCTTCCGATCT (SEQ ID NO: 5), 0.02 µM), and 1 µL of adapter seq2 primer (TACACTCTTTCCCTACACGACGCTCTTCCGATCTGACTCGTAATAC (SEQ ID NO: 6), 0.02 µM), corresponding to a part of the T7-promoter sequence)). This mix was amplifyied by PCR (98°C, 30 s; 15 cycles: 98°C 10 s; 65°C 75 s; then 65°C 5 min; 12°C hold) and then cleanned at 0.9X ratio with SPRlselect beads (Beckman Coulter B23318) and resuspended in 24 µL of ddH₂O.

A second PCR amplification was done by mixing the 24 µL of adaptors linked cDNA's complementary-probe strand with: 25 µL of Q5 hot start high fidelity 2X master mix, 0.5 µL of universal primer and 0.5 µL of index primer (0.1 µM final concentration NEBNext multiplex oligos for Illumina index primers set 1 E7335) by using the following cycling conditions: 98°C for 30s, 15 cycles of 98°C for 10s and 65°C for 75 s; then final extension at 65°C for 5 min and hold at 12°C.

Then sample was cleanned at 0.9X ratio with SPRlselect beads. At last the library was used for Illumina sequencing (150 nts paired-ends sequencing; NovaSeq).

### Bioinformatics processing

Primary analysis has been performed with our in-house developped tool SysISTD (SysFate Illumina Spatial transcriptomics Demultiplexer).

SysISTD takes as entry paired-end sequenced reads (fastq or fastq.gz format), and two TSV files, the first one containing the sequence of the molecular barcodes associated to the rows or column in the printed arrays and the second file presenting the physical position architecture of the spatial barcodes. SysISTD search for the Gibson sequence (regex query), then for two neighboring barcodes. Paired-reads presenting these features were aligned to either the human (hg19) or the mouse (mm9) genome with Bowtie2. In this manner, SysISTD generates SAM files per spatial coordinate, which are reprocessed with the help of featureCounts and an in-house script for detecting reads counts per gene promoter (+/-5kb) and per spatial coordinate (SysISPD (SysFate Illumina Spatial Promoters Demultiplexer)).

As outcome, SysISPD generates a matrix presenting read counts associated to physical coordinates in columns and known transcripts ID associated to the evaluated promoters in rows. To focus the downstream analysis to the physical positions corresponding to the analyzed tissue, it was used an in-house R script taking as entry an image of the DNA array scanned with the TRICT filter, releaving the presence of the fiducial borders.

Specifically, a croped image within the fiducials (imager package) was uploaded to R and the "px.flood" function was used to retrieve the pixels associated to the tissue. Finally it was applied a pixel to gexel coordinates conversion prior to cross this information with the outcome of SysISPD.

In parallel, all SAM files per coordinate were merged into a single-file and processed with the peak caller Macs2. The obtained confident peaks (P-value < 1x10⁻⁵) were annotated to their proximal coding regions. Finally, the spatial coordinates/promoters matrix provided by SysISPD was filtered by selecting transcripts associated to promoter regions presenting confident peaks.

The "confident promoter peaks tissue-focused" matrix was quantile normalized with our tool MULTILAYER. Then, the normalized matrix was further processed for revealing cell/tissue density contrast information by integrating normalized counts with pixel value levels retrieved in either the DAPI or the histone modification immunostaining image. The obtained normalized and pixels intensity adjusted matrix was used for all downstream analysis.

T-distributed stochastic neighbor embedding (t-SNE) analysis has been performed within R with the help of the Rtsne package. Projection of the stratified clusters (K-means metrics) was performed with the help of an in-house R script. Genes presenting over-represented counts per promoter and per cluster were used for performing a cell/tissue type enrichment analysis with EnrichR and by using the Mouse gene Atlas, Tabula Muris, ARCHS4, CellMarkerAugmented and Panglao databases.

### Mouse embryo sections alignment and chromatin state transitions detection

SpExel physical positions for each mouse embryo section were realigned relative to the tail of the embryo, allowing to associated the same pseudo-coordinates for all evaluated sections.

The associated histone modification identifier was added to the name of each transcript within the aligned matrices and concatenated into a single matrix allowing to interrogate for contiguous SpExels within sections and enrichment patterns similarity across sections in MULTILAYER. Contiguous promoter patterns and promoter co-enrichment patterns were captured by MULTILAYER over the embryo pseudo-map hosting data of all three histone modifications. Chromatin state transitions were computed by extracting promoter co-enrichment patterns similarity over all three sections with at least 5 consecutive SpExels.

### Example 1. A double-barcode spatial epigenomics strategy allowing to capture histone modification signatures across large tissue sections.

With the aim of capturing histone modifications across tissue sections, a DNA array manufacturing strategy has been engineered on the basis of the use of two complementary oligonucleotides printed at a high accuracy with the help of the pico-litter spotter Sciflexarrayer S3 (Scienion). First, oligonucleotides harboring an amino C6 linker modification at the 5' end for UV crosslinking, a T7promoter sequence, a unique molecular barcode per deposited row and a 30-nt-length adapter sequence, herein labelled as "Gibson", have been deposited as rows **(****Figure 1A****).** Then, on top of the previous deposited probes, the second set of oligonucleotides harboring a complementary Gibson sequence at the 3'-end, a unique molecular barcode per deposited column and a specific 19-nt transposase recognition sequence (Mosaic End, herein labelled as "MOS"), have been printed per columns.

Printed DNA arrays were first UV irradiated to crosslinking the DNA probes, then they were covered with a solution containing T4 DNA polymerase for elongating the hybridized probes, leading to a double-stranded DNA molecule harboring a unique combination of two molecular barcodes, as well as a MOS sequence at the exposed extremity of the DNA probe **(****Figure 1B****).** This strategy allows to manufacture DNA arrays composed by several thousands of DNA probes at a cost-effective manner since the molecular barcodes complexity is obtained by a combinatorial manner.

Tissue sections deposited on top of the double-barcoded DNA arrays were permeabilizied and incubated with an antibody against the histone modification of interest, followed by a secondary antibody binding allowing to enhance the tethering of the Protein A-Transposase Tn5 (PA-Tn5) **(****Figure 1C****).** A PA-Tn5 moeity devoid of loaded DNA sequences was used, such that the loading takes place with the MOS sequences retrieved in the exposed ends of the printed DNA probes **(****Figure 1D****).** In that manner, the cleaved chromatin in presence of Magnesium chloride remains captured by the proximal DNA probes **(****Figure 1E****).** After tissue degradation by proteinase K treatment, the DNA array was exposed to alkaline conditions (NaOH), revealing single-DNA ends for library preparation *in situ.* This was performed by extending DNA extremities by poly-C tailing with Terminal Transferase, hybridization of a poly-G primer presenting a known adapter sequence at its 5'-extremity (Adpt1), followed by Klenow Polymerase extension **(****Figure 1E****).** Finally, DNA arrays were exposed again to alkaline conditions for releasing the elongated complementary DNA sequence, and collected in a tube for performing two rounds of PCR amplification. The first PCR amplification step relies in a primer complementary to the T7-promoter sequence and presenting a known adapter fragment (Adpt2) as well as in a primer targeting the Adpt1. The second PCR amplification uses the Adpt1 and Adpt2 sequences for introducing the P5 and P7 illumina adapters, including a molecular barcode sequence, specific to the prepared library **(****Figure 1E****).**

This strategy has been validated in an adult mouse brain fresh-frozen tissue section deposited on top of a DNA array covering a surface of 16x8 mm, presenting a total of 4,096 unique combinations of dual barcodes **(****Figure 1F****).** The manufactured DNA array presented a pitch distance of 177 microns, with a DNA probe resolution of 100 microns (diameter spot) **(****Figure 1G****).** Tissue section has been immunolabelled with an antibody targeting the histone modification H3K4me3, known to be enriched in actively transcribing or bivalent promoter regions. For enhancing the tethering of the PA-Tn5, a secondary antibody has been added (1/100 dilution), complemented by the same secondary antibody conjugated to a fluorophore (AF-488) but loaded at a higher dilution level (1/1,000). In that manner, the performance of the histone modification labelling can be visualized in the same sample that will be used for the spatial epigenomics profiling **(****Figure 1H****).**

Once the immunolabelled mouse brain section was scanned for revealing the histone modification localization (FITC channel; **Figure 1H****)** as well as for capturing its physical position within the DNA array thanks to Cy3-labelled fiducial oligonucleotides deposited in the borders of the double-barcoded deposited probes (TRITC channel; **Figure 1F****),** the aforementioned procedure has been applied, leading to a sequencing library presenting DNA fragments ranging between 300 and 1,000 bp-length **(****Figure 1I****).** More than 250 million reads were Illumina sequenced and a custom-made bioinformatics pipeline has been applied, targeting the detection of the Gibson sequence, the presence of both unique molecular barcodes and the mapping to the mouse genome of the captured genomic sequence **(****Figure 1J****).** More than 40 million reads mapped to the mouse genome were processed as bulk with the peak caller Macs2, followed by the identification of promoter regions presenting confident peaks. Demultiplexing of these confident regions gave rise to ~2,000 read counts per SpExel (spatial Epigenomics element, in analogy to pixel for picture element), and ^{~}1,500 promoters per SpExel in average **(****Figure 1K****).**

Spatial epigenomic maps issued from confident peaks in promoters were first quantile normalized for correcting for potential technical variations across printed probes, then adjusted for cell density variation across the tissue by integrating immunostaining pixel information with the assesed read count levels. The obtained mouse brain spatial H3K4me3 epigenomic map revealed adjusted read counts fluctuating between 11 to 18 (log2 scale), in which the *corpus callosum* structure is visible due to its high adjusted read counts intensity **(****Figure 1L****).** Promoters enriched for the H3K4me3 histone modification across the spatial map were compared with those described in two public bulk epigenomic maps assessed on mouse brain samples, revealing 8,097 common genes, corresponding to > 60% of all retreived peaks in either conditions (bulk or spatial setting) **(****Figure 1M****).**

A t-distributed stochastic neighbor embedding (t-SNE) analysis stratified sPExels in 8 clusters, coincident with anatomical brain structures. For instance, H3K4me3 enrichment signatures retrieved in the gene promoter region coding for the Carbonyl reductase 3 (*Cbr3*)*,* or the Adenylyl cyclase 5 (*Adcy5*) were stratified within the same cluster (cluster 2). Furthermore, these genes were previously shown to be over-expressed in the mouse brain basal ganglia, concordant with the spatial signature revealed in the obtained H3K4me3 map **(****Figure 1N****)** as well as in the H3K4me3 spatial map published by Zhang et al. (Nature 616:113-122, 2023). Similarly H3K4me3 enrichment in the promoter region associated to the gene *Thap2* (THAP Domain Containing 2), but also *G6pc3* (Glucose-6-Phosphatase Catalytic Subunit 3) presented spatial signatures mainly concentrated around the *corpus callosum* structure in aggreement with the data provided in the H3K4me3 spatial map published by Zhang *et al.* **(****Figure 1N****).**

### Example 2. Major molecular players involded in rheumatoid arthritis can be detected by spatial epigenomics profiling performed in decalcified FFPE mouse paws samples

One of the major limitations of the available spatial transcriptomics technologies is their capacity to interrogate biological material in which the storage condition is not RNA-friendly. Specifically this corresponds to formalin-fixed, paraffin-embedded (FFPE) tissue samples, classically used for clinical sample preservation. While current spatial transcriptomics methods interrogates this type of samples by targeting the remaining RNA by hybridization, this strategy reaches its limits in bone-related samples where an acid-based decalcification procedure - prior FFPE embedding - further destroys the remaining RNA.

Considering that acid decalcified FFPE embedded samples are classically used for immunostaining assays after deparafination and rehydration, it was thought that the present spatial epigenomics strategy should be able to capture histone modification enriched chromatin. To address the performance of the spatial epigenomics methodology of the invention in bone-related FFPE samples, samples issued from a collagen-induced arthritis (CIA) mouse model were used. Specifically, CIA mouse paws were fixed with paraformaldehyde, decalcified in presence of formic acid and FFPE embedded **(****Figure 2A****).** FFPE blocks were cut to obtain thin sections that were deposited on top of the manufactured DNA arrays harboring mosaic sequences. DNA arrays were deparaffined and rehydrated by following standard procedures, followed by spatial epigenomics processing **(****Figure 2A****).**

Sagittal sectioning of the mouse paws allows to evaluate the ankle and tarsal joints, visualized by the presence of a sagittal plane of the talus, navicular, cuneiform and meta-tarsus bones **(****Figure 2B****).** To capture potential actively transcribing genes a spatial epigenomics profiling targeting the histone modification H3K27 acethylation (H3K27ac) over a DNA array composed of 2,048 unique double-barcoded DNA probes was performed **(****Figure 2C****).** Illumina sequencing of the obtained spatial epigenomics library provided > 330 million reads, from wich ~77% presented the Gibson sequence and ~40% presented in addition the dual barcodes **(****Figure 2D****).** Mapping to the mouse reference genome, allowed to identify ~78 million reads, which were processed as bulk for identifying confident peaks within promoter regions. Demultiplexing of these confident regions gave rise to ~2,000 read counts per SpExel and ~1,800 promoters per SpExel in average **(****Figure 2E****).**

H3K37ac spatial epigenomic map issued from confident peaks in promoters was quantile normalized and adjusted for variations in tissue density, leading to a digital map representing the ajusted read counts per physical position across the tissue **(****Figure 2F****).** A t-SNE analysis stratified the corresponding SpExels in 6 clusters, which were then projected on the spatial tissue map **(****Figure 2G****&****H****).** Tissue-cell types gene marker association analysis allowed to identify major relevant terms corresponding to bone composition (Mesenchymal progenitors: clusters 1&6; Osteocytes: clusters 1,2&3; Osteoblasts: clusters 2&6), cartilage (cartilage progenitor cell: cluster 1&6; mesenchymal progenitors: cluster 6), as well as synovial joint (Mesenchymal stem cells: Synovial fluid: cluster 4) **(****Figure 2I****).** Furthermore, Tissue-cell types gene markers associated to an inflammatory response were detected, including the presence of regulatory T cell signatures (FOXP3+ Natural Regulatory T(Treg) cells, Monocytes: cluster 4&5; large granular lymphocytes: cluster 6; Regulatory T cells: cluster 1,4,5&6); as well as markers assocaited to osteoarthritic cartilage tissue (Mesenchymal progenitors: cluster 4) **(****Figure 2I****).**

Notably, H3K27ac enrichment signatures in promoters associated to genes coding for proteins like the transmembrane glycoprotein podoplanin (Pdpn) - known to participate in osteocyte function, but also as an important sensor for bone damage during inflammatory arthritis - or the Fibroblast growth factor 23 (FGF23) mainly produced by osteocytes and in elevated levels in rheumatoid arthritis, were observed across the mouse paws sections **(****Figure 2J****).** Similarly, promoter regions associated to genes like *Dlx5* (Distal-Less Homeobox 5) - a well known transcription factor driving osteoblastogenesis, or *GABBR1* (Gamma-Aminobutyric Acid Type B Receptor Subunit 1), a member of the GABA receptors and known to be over-expressed in rheumatoid arthritis, were also retrieved enriched in H3K27ac signatures across the tissue.

Furthermore, promoter regions associated to genes coding for pro-inflammatory cytokines including IL1b, IL6 or IL10 - known to drive inflammation and the destructive process of the tissue - the cytokines CD44, CD166, CD248, the colony-stimulating factor (CSF1) - driving the development of the monocyte-macrophage cell lineage, which play an important function in the pathogenesis of rheumatoid arthritis, or the Integrin Subunit Alpha 2 (ITGA2) - shown to be over-expressed in RA and influencing T cell growth accompanied by an upregulation of pro-inflammatory cytokine expression - were also enriched for H3K27ac across the studied CIA mouse paws **(****Figure 2J****).**

Overall, the aforementioned data clearly demonstrate the capacity of the double-barcoded spatial epigenomics strategy to retrieve RA-related signatures from acid-decalcified FFPE mouse paws samples.

### Example 3. Spatially resolving chromatin state signatures by integrating multiple histone modification maps issued from consecutive mouse embryo sections.

To further illustrate the performance of the presented technology, spatial epigenomics profiling has been performed over mouse embryo sections (E11.5). DNA arrays composed by 32x32x2 unique double-barcoded probes (32x32 interstitially printed arrays; 2,048 spots) were manufactured. Mouse embryo sections deposited on top of the DNA arrays were immunolabelled for the histone modification H3K27ac, known to be enriched in active promoters as well as in active enhancer regions. DAPI staining has been used for revealing cell density variability across the tissue section, which has been integrated within the read counts normalization **(****Figure 3A****).** A t-SNE analysis revealed 6 clusters presenting distinct anatomical locations when projecting this information across the spatial SpExels locations **(****Figure 3B****).** Tissue-associated gene ontology enrichment analysis confirmed the anatomical classification, illustrated by the spinal cord signature retrieved in cluster 2&5; the epicardial progenitor cell hearth association in cluster 1&4; a strong nervous tissue association in cluster 5; the presence of olfactory ensheathing cell signatures in cluster 3, known to be derived from neural crest cells and detectable by the expression of *Sox10* at E11.5; or a ventral otocyst brain signature in cluster 6&4; corresponding to progenitor cells of the inner ear deteactable at E10.5 thanks to the expression of the transcription factor *Pax2* **(****Figure 3C****).**

In addition to the H3K27ac local enrichment in the promoter regions of *Sox10* or *Pax2,* other genes like *Lhx5* (LIM Homeobox 5), known to be broadly expressed in the early embryo but later restricted to the nervous system; the transmembrane protein 80 (T*mem80*), previously shown to be expressed in the head surface ectoderm (RNA-seq assays in embryonic mice (E8.5, E9.5, E10.5)), or two members of the Wnt familly: *Wnt4,* previously shown to be expressed in the spinal cord of E9.5 and E10.5 mouse embryos; or *Wnt11,* shown to be required for proper patterning of the neural tube and somites by regulating notochord formation were also enriched for this histone modification **(****Figure 3D****).** The relevance of finding H3K27ac enrichment in the aforementioned gene promoters was also confirmed in the spatial epigenomics cartography previously described by Deng et al. (Science 375:681-686, 2022), but also by gene expression data assessed in mouse embryo (E11.5) by the Allen developping mouse brain atlas **(****Figure 3E****).**

While several of the retrieved signatures in the spatial H3K27ac cartography do match with previously described gene expression patterns, this histone modification alone is not a fateful marker of active transcription. Indeed, previous studies demonstrated a correlative relationship between the presence of co-occurent histone modifications at promoter regions with the transcriptional status of the associated gene. For instance, promoter regions hosting both H3K4me3 and H3K27ac signatures are known as markers for active transcription, while promoters enriched on H3K27me3 correspond to repressed regions. Furthermore, promoters hosting the H3K27me3 modification and either H3K4me3 or H3K27ac are described as "bivalent promoters", corresponding to genes devoid of transcription acitivity, but prone to initiate gene expression. Herein, it was aimed to evaluate this potential combinatorial co-enrichment signatures (previously described as chromatin state analysis) for three major histone modifications: H3K4me3, H3K27ac and H3K27me3. Hence, in addition to the aforementioned H3K27ac spatial cartography, spatial epigenomics maps were also generated for H3K4me3 and H3K27me3 issued from consecutive sections **(****Figure 3F****).** Since mouse embryo sections are not located in exactly the same physical position on the manufactured DNA arrays, all three spatial epigenomic maps were computationally realigned, leading to the generation of a spatial pseudo-map, used in a second time for interrogating histone enrichment signatures per section, but also across all three overlayed sections **(****Figure 3G&H****).** The obtained pseudo map has been first used to verify the spatial co-ehrichment of the studied histone modifications in the case of the gene *Wnt11.* While all three histone modifications were retrieved over-enriched in *Wnt11* promoter regions **(****Figure 3D****&****F****),** they do not systematically overlay in their spatial localizations within the embryo, as revealed when interrogating for contiguous over-enriched SpExels (spatial epigenomics elements) within the pseudo-map. Indeed, distinct spatial localizations within the embryo appeared to be enriched for both H3K4me3 and H3K27ac or the repressive mark H3K27me3 **(****Figure 3I****).** This is further confirmed when interrogating for *Wnt11* co-enrichment promoter signatures (i.e. other genes presenting the same histone modification erichments in their promoters and spatially colocalized with *Wnt11*)*.* Wnt11 H3K27ac/H3K4me3 (active promoter signature) co-enrichment patterns were associated to the trunk notochord region, while the repressive mark H3K27me3 appears to be enriched to the tail of the embryo, **(****Figure 3J****),** in aggreement with *Wnt11* expression described in previous studies.

Expanding the spatial co-enrichment analysis over all genes captured in the spatial epigenomic maps revealed > 600 promoters presenting H3K4me3/H3K27ac co-enrichment signatures (active promoters), 4,759 repressed promoters (enriched for H3K27me3) and > 1,600 bivalent promoters; defined by their enrichment in H3K27me3 and either H3K4me3 (State 4: 681 genes), or H3K27ac (State 2: 974 genes) **(****Figure 3K****).** Finally, a large fraction of gene promoters were retrieved spatially associated only to H3K4me3 (3,500) or H3K27ac (5,140), potentially indicating that it was failed to properly associate them to a co-enrichment signature due to technical issues (e.g. > 5 contiguous over-enriched SpExels; sequencing depth; antibody performance; etc).

As illustrated for the case of the Wnt11 promoter **(****Figure 3I & 3J****),** chromatin states in promoter regions do change across tissue section. To illustrate these changes, some genes have been selected on the basis of the number of over-enriched SpExels across the tissue (>40% when considering all chromatin state combinations; and >10% for at least one of the chromatin states). A total of 750 genes were selected, revealing 291 promoters fluctuating between the active to the repressive state, passing by the bivalent situations. Furthermore, the remaining 459 genes were devoid of the active chromatin state combination, but presented the repressive mark H3K27me3 **(****Figure 3L****).** In some cases these promoters shifted to either of the bivalent states (62 genes) or replaced the repressive mark by either H3K4me3 or H3K27ac (80 genes), or they fluctuated between only one of the bivalent states and the presence of only H3K4me3 or H3K27ac (143 genes hosting the chromatin state S2, 102 genes hosting the S4). Finally, there are 72 genes that do not present the H3K4me3 alone situation, but fluctuates between the repressive state (H3K27me3), the bivalent state S2 (H3K27me3/H3K27ac) or the presence of only the histone modification H3K27ac in their promoter regions **(****Figure 3L****).** The fact that these 459 gene promoters are devoid of the active chromatin state, but at the same time do present either H3K4me3 or H3k27ac alone, do support the possibility of missing such signature due to technical limitations. For instance, in the case of the *Hoxb4* promoter, the present spatial epigenomics profiling clearly revealed a strong repressive signature (H3K27me3) other than the spinal cord region. In contrary, the H3K4me3 and H3K27ac profiling do present a spinal cord enrichment localization **(****Figure 3M****).** *Hoxb4* gene co-enrichment similarity maps revealed a bivalent chromatin state configuration at the tail of the embryo, and a coenrichment signature of the histone modifications H3K4me3 and H3K27ac at the medial level of the spinal cord **(****Figure 3M****),** in agreement with gene expression revealed by in situ hybridization (Allen developping mouse brain atlas; **Figure 3N****).**

Overall, this complex combinatorial chromatin state transitions clearly reflects tissue complexity, but also provide means to deeply interrogate histone modification signature transitions across the developping system. It is clear, that having multiple histone modification profiles and ideally multiple consecutive sections appears essential for further exploiting this molecular cartography.

## Claims

1. A DNA array exposing a set of barcoded DNA polynucleotides, wherein each polynucleotide is immobilized by one of its ends to the array, wherein:
each polynucleotide comprises:
- a terminal region, at the end attached to the array,
- a region for spatial barcoding,
- a mosaic region (MOS), at the free end, said MOS region being recognized by a transposase,
and each distinct polynucleotide occupying a distinct position in a grid pattern corresponding to its spatial barcoding.

2. A DNA array according claim 1, wherein the region for spatial barcoding comprises:
- a region for spatial barcoding assigned to a specific row of the array (BCr), and
- a region for spatial barcoding assigned to a specific column of the array (BCc),
and each distinct polynucleotide occupies a distinct position in a grid pattern corresponding to its BCr and BCc regions.

3. A method comprising:
(i) depositing a tissue section on top of a DNA array as defined in claim 1 or 2,
(ii) delivering to the DNA array a primary antibody that specifically binds an epigenomic marker of interest, and possibly a secondary antibody that binds the first antibody, optionally said primary antibody and/or secondary antibody presenting a conjugated fluorophore,
(iii) delivering to the DNA array a fusion protein which comprises a moiety that binds the primary antibody, or the secondary antibody if present, and a moiety that is a transposase recognizing the MOS region of the barcoded DNA polynucleotides,
(iv) incubating the DNA array in conditions allowing the cleavage of the genomic DNA in the tissue section and its ligation with the MOS region of the barcoded DNA polynucleotides by the transposase, preferably in presence of MgCl₂,
(v) incubating the DNA array with a protease, preferably proteinase K.

4. A method according to claim 3, further comprising:
(vi) copying the DNA captured by the barcoded DNA polynucleotides.

5. A method according to any one of claims 3 or 4, further comprising:
(vii) sequencing the DNA captured by the barcoded DNA polynucleotides to produce DNA reads.

6. A method according to any one of claims 3 to 5, further comprising:
(viii) constructing a spatial map of the tissue sample by matching the barcoded nucleotides to corresponding sequencing reads.

7. A method according to any one of claims 3 to 6, further comprising:
(ix) identifying an anatomical location of the DNA captured by the barcoded DNA polynucleotides in the tissue sample.

8. A method according to any one of claims 3 to 7, wherein the fusion protein is composed by a transposase and a protein A moiety.

9. A method according to any one of claims 3 to 8, wherein the epigenomic marker of interest is selected from the group consisting of H2AK5ac, H2AK9ac, H2BK120ac, H2BK12ac, H2BK15ac, H2BK20ac, H2BK5ac, H2Bub, H3, H3ac, H3K14ac, H3K18ac, H3K23ac, H3K23me2, H3K27me1, H3K27me2, H3K36ac, H3K36me1, H3K36me2, H3K4ac, H3K56ac, H3K79me1, H3K79me3, H3K9acS10ph, H3K9me2, H3S10ph, H3T11ph, H4, H4ac, H4K12ac, H4K16ac, H4K5ac, H4K8ac, H4K91ac, H3F3A, H3K27me3, H3K36me3, H3K4me1, H3K79me2, H3K9me1, H3K9me2, H3K9me3, H4K20me1, H2AFZ, H3K27ac, H3K4me2, H3K4me3, H3K9ac, and any other histone modification.

10. Use of a DNA array as defined in claim 1 or 2 for spatial mapping of an epigenomic marker of interest.

11. A method of manufacturing a DNA array as defined in claim 1 or 2, comprising immobilizing DNA polynucleotides to the array, each polynucleotide comprising:
- a terminal region, at the end attached to the array,
- a region for spatial barcoding,
- a mosaic region (MOS), at the free end, said MOS region being recognized by a transposase,
and each distinct polynucleotide being immobilized at a distinct position in a grid pattern corresponding to its spatial barcoding.

12. A method of manufacturing a DNA array as defined in claim 1 or 2, comprising:
(i) immobilizing single stranded DNA polynucleotides by their 5' end to the array, each polynucleotide comprising from 5' end to 3' end:
- a terminal region,
- a region for spatial barcoding assigned to a specific row of the array (BCr),
- a bridge region,
each polynucleotide being positioned at a distinct position in a grid pattern corresponding to its BCr region,
(ii) delivering at the surface of the array single stranded DNA polynucleotides, each polynucleotide comprising from 5' end to 3' end:
- a mosaic region (MOS) being recognized by a transposase,
- a region for spatial barcoding assigned to a specific column of the array (BCc),
- a bridge region, which is complementary of the bridge region of the polynucleotides immobilized on the array,
each polynucleotide being positioned a distinct position in a grid pattern corresponding to its BCc region,
(iii) hybridizing and elongating the polynucleotides by a DNA polymerase, preferably a T4 DNA polymerase.

13. A DNA polynucleotide comprising:
- a terminal region, at one end
- a region for spatial barcoding,
- a mosaic region (MOS), at the other end, said MOS region being recognized by a transposase.

14. A set of two groups of DNA polynucleotides, wherein :
- the first group of DNA polynucleotides comprises from 5' end to 3' end:
o a terminal region,
o a region for spatial barcoding,
o a bridge region, and
- the second group of DNA polynucleotides comprises from 5' end to 3' end:
o a mosaic region (MOS) being recognized by a transposase,
o a region for spatial barcoding,
o a bridge region, which is complementary of the bridge region of the DNA polynucleotide of the first group.

15. A kit comprising at least:
- a DNA array as defined in claims 1 or 2,
- a DNA polynucleotide as defined in claim 13, or
- a set of two groups of DNA polynucleotides as defined in claim 14.
